**Europäisches Patentamt**

**European Patent Office**

**Office européen des brevets**

(19)

(11) Veröffentlichungsnummer: **0 017 180**
A2

(12)

# EUROPÄISCHE PATENTANMELDUNG

(21) Anmeldenummer: **80101650.2**

(22) Anmeldetag: **27.03.80**

(51) Int. Cl.³: **C 07 C 126/02**

(30) Priorität: **02.04.79 DE 2913179**

(43) Veröffentlichungstag der Anmeldung:
**15.10.80 Patentblatt 80/21**

(84) Benannte Vertragsstaaten:
**AT BE CH DE FR GB IT LU NL SE**

(71) Anmelder: **BASF Aktiengesellschaft
Carl-Bosch-Strasse 38
D-6700 Ludwigshafen(DE)**

(72) Erfinder: **Luetzow, Dietrich, Dr. Chem.
Vischerstrasse 14
D-6703 Limburgerhof(DE)**

(72) Erfinder: **Struve, Herbert, Dr. Dipl.-Ing.
Oelbergstrasse 26
D-7840 Muellheim-Niederweiler(DE)**

(54) **Anordnung am Verdichter einer Harnstoff-Syntheseanlage.**

(57) Die Erfindung betrifft eine Anordnung am Verdichter einer Harnstoff-Syntheseanlage, aus der gasförmiges überschüssiges Ammoniak fortlaufend über einen Kondensator in einen unter Druck gehaltenen Vorratsbehälter zurückgeführt und aus diesem unter gewisser Druckerhöhung wieder der Saugseite des mit ölgeschmierten Stopfbüchsen betriebenen Kolbenverdichters zugeführt wird, wobei die Entlüftungsleitung der Stopfbuchsen zur besseren Ausschleusung von Ölspuren über einen Ölabscheider und über die das zurückgeführte Ammoniak aufnehmende Seite des Kondensators mittelbar mit dem Vorratsbehälter verbunden ist.

EP 0 017 180 A2

BASF Aktiengesellschaft                    O.Z. 0050/033/73

## Anordnung am Verdichter einer Harnstoff-Syntheseanlage

Die Erfindung bezieht sich auf Harnstoff-Syntheseanlagen, an deren Eingangsseite Ammoniak im flüssigen Zustand aus einem unter Drucken von etwa 15 bis 25 bar gehaltenen Lagerbehälter fortlaufend entnommen und anschließend mittels eines oder mehrerer Kolbenverdichter auf höhere Drucke gebracht wird, um dann in die Synthese einzutreten. Hierbei sind besondere Vorkehrungen zu treffen, um das Ammoniak an den Kolben- und Stopfbüchsen der Verdichtermaschinen vor Verunreinigungen mit Schmierstoffen zu schützen. Bei den hohen Reinheitsanforderungen, die heute an technische Endprodukte gestellt werden, wirkt oft schon ein Gehalt an wenigen ppm Öl störend, z. B. durch deutliche Opaleszens der wäßrigen Harnstofflösungen.

Es ist daher üblich, in Fällen besonders hoher Reinheitsforderungen trockenlaufende Stopfbüchsen zur Abdichtung der Plunger vorzusehen, um eine Verunreinigung des Produkts durch Schmierstoffe auszuschließen. - Es werden andererseits durch ein Schmier- und Sperrölsystem für die Stopfbüchsen erheblich längere Laufzeiten der Maschinen erzielt. Wird für eine Großanlage allgemein eine längere mittlere Betriebsdauer ohne Abstellungen für Wartungs- und Überholungsarbeiten gefordert, so ist man praktisch gezwungen, nasslaufende, d.h. an ein Schmiersystem angeschlossene Stopfbüchsen einzurichten und besondere Maßnahmen zu treffen, um eine Verunreinigung des Produktes zu vermeiden. Diese zusätzlichen besonderen Maßnahmen beinhalten z.B. das Filtern der verunreinigten Lösungen, - auch unter Zusatz von Aktivkohle oder das Ausscheiden von Kristallen aus der Lösung mit anschließender Wäsche zur Entfernung der verschmutzten Mutterlauge. Derartige aufwendige Maßnahmen erbringen dennoch häufig nicht den

F/BL

gewünschten Erfolg, nämlich die Herstellung einer wäßrigen Harnstofflösung, die keine Opaleszens zeigt.

An Kolbenverdichtern ist es aus betrieblichen und aus Sicherheitsgründen erforderlich, die Entlüftungsleitung der Hauptstopfbüchsen mit der Saugseite der Kolbenmaschinen zu verbinden, bzw. den Raum zwischen der Vor- und der Hauptstopfbüchse eines Verdichters über eine Leitung an den Saugstutzen der Maschine anzuschließen. Außer der abzuscheidenden Leckölmenge, die den Produktstrom zur Saugseite der Maschine hin nicht verunreinigen soll, ist zusätzlich stets eine gewisse Leckölmenge gegeben, die aus der Vorstopfbüchse des Kolbenverdichters ins Freie gelangt. Die letztgenannte Leckölmenge ist unter anderem abhängig von der Größe des absoluten Drucks auf der Saugseite der Kolbenmaschine.—Das zu verdichtende Ammoniak wird an der Eingangsseite der Harnstoff-Syntheseanlage einen unter Drucken von etwa 10 bis 20 bar gehaltenen Lagertank oder einem anderem Vorratsbehälter entnommen und der Kolbenmaschine mittels einer Kreiselpumpe zugeführt, die den Druck auf etwa 15 bis 25 bar anhebt.

Es wurde nun gefunden, daß man bei einem Sperröleinsatz zur Schmierung der Stopfbüchsen der Ammoniak-Kolbenverdichter die Produktverunreinigung wesentlich verringern und zusätzlich die Leckmenge an Öl und Ammoniak aus der Vorstopfbüchse des Kolbenverdichters ins Freie verringern kann, wenn die Entlüftungsleitung der Verdichter-Stopfbüchen über einen Ölabscheider und über die das zurückgeführte Ammoniak aufnehmende Seite des Kondensators mittelbar mit dem Vorratsbehälter verbunden ist. - Nach einem weiteren Merkmal der Erfindung ist der Dampfraum des Ölabscheiders unter einem dem Rückführsystem für überschüssiges gasförmiges Ammoniak entsprechenden Druck gehalten.

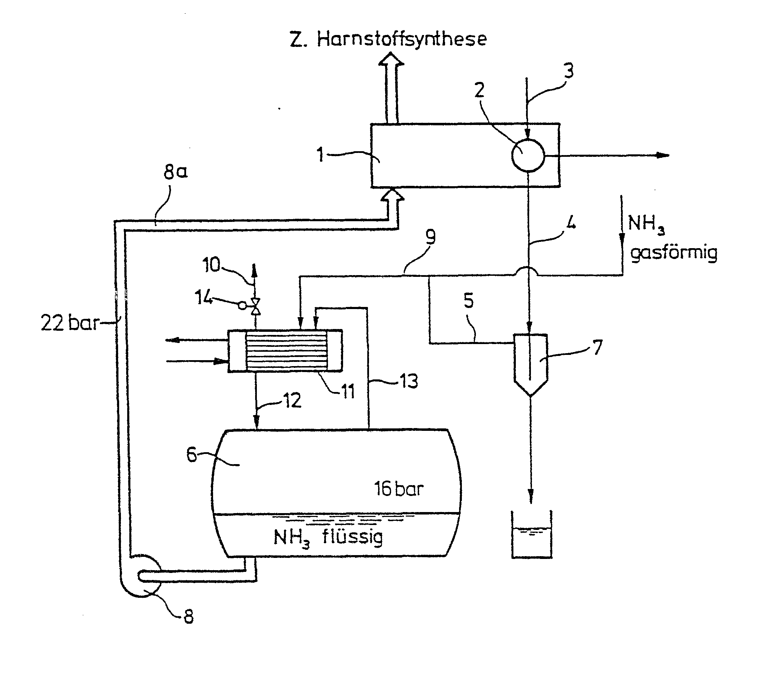

In der Zeichnung ist schematisch die Eingangsseite einer Harnstoff-Syntheseanlage bis zur Kolbenverdichtermaschine 1 wiedergegeben. Das Stopfbüchsensystem 2 des Plungers ist an ein Drucköisystem 3 angeschlossen. Das Schmieröl bzw. Sperröl gewährleistet ferner die Feinabdichtung der Stopfbüchse gegenüber dem zu verdichtenden flüssigen Ammoniak. Das aus der Syntheseanlage fortlaufend zurückgeführte gasförmige Ammoniak gelangt bei Drucken von etwa 10 bis 20 bar über die Leitung 9 zum Kondensator 11 und tritt über die Leitung 12 in flüssigem Zustand in den Vorratsbehälter 6 ein. In diesem Vorratsbehälter herrscht ebenfalls ein Druck zwischen etwa 10 bis 20 bar, wobei aus seinem Dampfraum austretende Restmengen gasförmigen Ammoniaks über die Leitung 13 nochmals zum Kondensator 11 zurückgeführt werden können. Das ganze in einem solchen Druckbereich gehaltene System kann zur Aufrechterhaltung eines bestimmten Druckes von z.B. 16 bar mittels des Regelventils 14 über die Leitung 10 geregelt entspannt werden.

Die Entlüftungsleitung 4 der Stopfbüchse 2 der Kolbenmaschine ist nicht, wie es sonst bei Kolbenmaschinen üblich ist, mit der Saugseite verbunden. Vielmehr ist erfindungsgemäß die Entlüftungsleitung 4 der Verdichterstopfbüchsen 2 über einen Ölabscheider 7 und mittels der Verbindungsleitung 5, über die das zurückgeführte Ammoniak aufnehmende Seite des Kondensators 11, mittelbar mit dem Vorratsbehälter 6 verbunden. Mittels der Kreiselpumpe 8 wird in der Ansaugleitung 8a des Kolbenverdichters ein Druck von etwa 20 bis 25 bar aufrechterhalten. Da an der Stopfbüchse 2 der im Vorratsbehälter 6 herrschende niedere Druck von etwa 15 bis 20 bar, vorzugsweise ein solcher von 16 bar anliegt, wird bei dieser Anordnung gleichzeitig auch das Austreten von gasförmigem Ammoniak

aus der Stopfbüchse 2 ins Freie wesentlich verringert. Aufgrund der solcher Art gegebenen Druckdifferenzen auf der Rückführungsseite des gasförmigen Ammoniaks bis zum Vorratsbehälter 6 einerseits und in der Ansaugleitung 8a unter der Kreiselpumpe 8 andererseits erfolgt im Abscheider 7 eine destillative Abtrennung des Ammoniak-Leckanteils aus der Emulsion $NH_3$/Öl ohne zusätzlichen Aufwand. Das ausgeschleuste Öl ist, eventuell nach einer einfachen Wasserwäsche, erneut als Sperr-bzw. Schmieröl einsetzbar.

Beispiel 1

Bei der Herstellung von Harnstoff aus $CO_2$ und $NH_3$ wird das $NH_3$ flüssig mit Kolbenpumpen auf den Reaktionsdruck von 150 bis 300 atü verdichtet. Die Stopfbüchsen dieser Pumpen sind zur Verlängerung der Plunger-Standzeiten und Verbesserung der Stopfbuchsenabdichtung mit einem Sperr-/Schmierölsystem ausgerüstet. Dadurch gelangt zusätzlich Öl in die Syntheseanlage; die so gewonnene Harnstofflösung weist einen Gesamtölgehalt von 25 bis 30 ppm auf. - Aus dieser Lösung lassen sich auch nach einer Filtration keine Harnstoffkristalle herstellen, die sich klar in Wasser lösen. Die Lösungen weisen Trübungswerte auf, die denen von Testlösungen mit über 15 ppm $SiO_2$ pro Liter entsprechen.

Beispiel 2

Verzichtet man darauf, die Stopfbüchsen mit einem Sperr-Schmierölsystem auszurüsten, um ölärmere Harnstofflösungen zu erhalten, so sinkt die Standzeit der Plunger auf 20 bis 30 % der gemäß Beispiel 1 erreichbaren Zeiten. Daneben stört die stärkere Belästigung im Arbeitsbereich durch den aus den Stopfbüchsen austretenden $NH_3$-Leckanteil.

Beispiel 3

Setzt man der gemäß dem Beispiel 1 hergestellten Harnstofflösung vor der Filtration Aktivkohle zu, so läßt sich der obige Ölgehalt auf etwa die Hälfte senken. Auch aus dieser Lösung hergestellte Kristalle lösen sich nicht klar, es bleibt eine deutliche Opaleszenz, die Trübungswerte liegen bei 7 bis 15 ppm $SiO_2$.

Beispiel 4

Wird Harnstofflösung nach dem im Anspruch genannten Verfahren hergestellt, d.h. das flüssige $NH_3$ beim Verdichten nicht mit Öl verunreinigt, so erhält man beim Weiterverarbeiten der Lösung nach Beispiel 2 Harnstoffkristalle, die sich klar in Wasser lösen. Die Trübungswerte liegen bei 1 bis 2 ppm $SiO_2$, obgleich der Aktivkohlezusatz gegenüber Beispiel 2 auf die Hälfte reduziert worden war. - Durch das Ausschleusen des Öls beim Pumpen wird die Bildung von stark dispergierten und emulgierten Ölanteilen verhindert. - Das Öl läßt sich mit der erfindungsgemäßen Anordnung leicht von dem $NH_3$-Leckanteil abtrennen; das $NH_3$ wird durch die Verbindung der Zwischenstopfbüchse mit einem auf niedrigerem Druck befindlichen $NH_3$-Lagertank praktisch destillativ entfernt.

BASF Aktiengesellschaft — 5 — O.Z.0050/033773

Patentansprüche

1. Anordnung am Verdichter einer Harnstoff-Syntheseanlage, aus der gasförmiges überschüssiges Ammoniak fortlaufend über einen Kondensator in einen unter Druck gehaltenen Vorratsbehälter zurückgeführt und aus dem Vorratsbehälter mittels einer Förderpumpe unter gewisser Druckerhöhung wieder der Saugseite des mit geschmierten und entlüfteten Stopfbüchsen betriebenen Kolbenverdichters zugeführt wird, dadurch gekennzeichnet, daß die Entlüftungsleitung (4) der Verdichterstopfbuchsen (2) über einen Ölabscheider (7) und über die das zurückgeführte Ammoniak aufnehmende Seite des Kondensators (11) mittelbar mit dem Vorratsbehälter (6) verbunden ist.

2. Anordnung nach Anspruch 1, dadurch gekennzeichnet, daß der Dampfraum des Ölabscheiders (7) unter einem dem Rückführsystem für überschüssiges gasförmiges Ammoniak entsprechenden Druck gehalten ist, der niedriger liegt als der Druck in der Ansaugleitung (8a) des Kolbenverdichters (1).

Zeichn.

$\frac{1}{1}$

Z. Harnstoffsynthese

NH$_3$ gasförmig

22 bar

16 bar

NH$_3$ flüssig

34/